# EUROPÄISCHE PATENTANMELDUNG

(11) **EP 3 748 644 A1**
(43) Veröffentlichungstag der Anmeldung: **09.12.2020**
(21) Anmeldenummer: 20176449.5
(22) Anmeldetag: 26.05.2020
(51) Int. Cl.: G16H 40/60

(54) **ANZEIGESYSTEM UND VERFAHREN ZUM ANZEIGEN EINER AUSGABE EINES ELEKTROMEDIZINISCHEN GERÄTES**

(30) Priorität: 07.06.2019 DE 102019003996
(71) Anmelder: Drägerwerk AG & Co. KGaA, 23558 Lübeck (DE)
(72) Erfinder: Schlichting, Stefan, 23558 Lübeck (DE); Scheuplein, Sina, 23558 Lübeck (DE)

(57) **Zusammenfassung**

Die Erfindung betrifft ein Anzeigesystem (100) zum Anzeigen einer Ausgabe mindestens eines elektromedizinischen Gerätes (105), mit einer Anzeigeeinheit (110), einer Eingabeeinheit (120), einer Anzeigesignaleinheit (130) und einer Datenverarbeitungseinheit (140). Die Anzeigeeinheit ist ausgebildet, ein erstes Anzeigesignal (112) und ein zweites Anzeigesignal (114) zu empfangen und basierend auf den beiden Anzeigesignalen eine kombinierte visuelle Ausgabe (116) darzustellen. Die Anzeigesignaleinheit weist eine Kommunikationsschnittstelle (132) zur Kommunikation mit dem mindestens einen elektromedizinischen Gerät auf und ist ausgebildet, Geräte-Daten (107), die das elektromedizinische Gerät betreffen, über die Kommunikationsschnittstelle zu empfangen, diese Geräte-Daten einem Visualisierungstyp zuzuordnen und abhängig von den aktuell darzustellenden Visualisierungstypen eine jeweilige Darstellungsart der empfangenen Geräte-Daten dynamisch zu bestimmen und darauf basierend das erste Anzeigesignal (112) zu erzeugen und auszugeben. Die Datenverarbeitungseinheit ist ausgebildet, Datensatz-Daten (142) auszulesen und aus diesen Datensatz-Daten das zweite Anzeigesignal (114) zu erzeugen und auszugeben, wobei eine Anmeldemaske (144) ausgeben wird, falls für aktuell ausgelesene Datensatz-Daten aus einer vorbestimmte Teilmenge von Datenarten nicht aktuell ein diesen Datenarten zugeordneter Berechtigungszustand des Anzeigesystems vorliegt.

## Beschreibung

Die Erfindung betrifft ein Anzeigesystem zum Anzeigen einer Ausgabe mindestens eines elektromedizinischen Gerätes und ein entsprechendes Verfahren zum Anzeigen einer Ausgabe mindestens eines elektromedizinischen Gerätes. Weiterhin betrifft die Erfindung ein Programm mit einem Programmcode zur Durchführung des erfindungsgemäßen Verfahrens.

Im medizinischen Umfeld ist die Verwendung einer Anzeigeeinheit bekannt, die die Daten eines angeschlossenen elektromedizinischen Gerätes anzeigt und mit dem Betriebsmodus dieses elektromedizinischen Gerätes synchronisiert ist.

US 9,104,789 B2 lehrt, dass eine Anzeigeeinheit Daten von verschiedenen angeschlossenen elektromedizinischen Geräten empfängt und variabel darstellt. Dabei werden Alarminformationen in einem separaten, expandierten Fenster eines Steuermenüs der Anzeigeeinheit dargestellt.

US 2016/0128647 A1 beschreibt ein medizinisches Anzeigesystem, das empfangene Daten von verschiedenen angeschlossenen elektromedizinischen Geräten empfängt und nach ihrer Wichtigkeit sortiert ausgibt.

Aufgabe der vorliegenden Erfindung ist es, eine verbesserte Ausgabe mindestens eines elektromedizinischen Gerätes, insbesondere eine mit anderen Geräten kompatible Ausgabe von Daten mindestens eines elektromedizinischen Gerätes, bereitzustellen.

Erfindungsgemäß wird zur Lösung dieser Aufgabe ein Anzeigesystem zum Anzeigen einer Ausgabe mindestens eines elektromedizinischen Gerätes vorgeschlagen, mit einer Anzeigeeinheit, einer Eingabeeinheit, einer Anzeigesignaleinheit und einer Datenverarbeitungseinheit.

Die Anzeigeeinheit ist ausgebildet, ein erstes Anzeigesignal und mindestens ein zweites Anzeigesignal zu empfangen und basierend auf den beiden Anzeigesignalen eine kombinierte visuelle Ausgabe darzustellen.

Die Eingabeeinheit weist eine Benutzerschnittstelle auf, über die ein Nutzer eine Benutzereingabe bereitstellen kann.

Die Anzeigesignaleinheit weist eine Kommunikationsschnittstelle zur Kommunikation mit dem mindestens einen elektromedizinischen Gerät auf und ist ausgebildet, Geräte-Daten, die das elektromedizinische Gerät betreffen, über die Kommunikationsschnittstelle zu empfangen, diese Geräte-Daten einem Visualisierungstyp zuzuordnen und abhängig von den aktuell darzustellenden Visualisierungstypen eine jeweilige Darstellungsart der empfangenen Geräte-Daten dynamisch zu bestimmen. Weiterhin ist die Anzeigesignaleinheit ausgebildet, basierend auf den Geräte-Daten und der jeweils bestimmten Darstellungsart das erste Anzeigesignal zu erzeugen und an die Anzeigeeinheit auszugeben.

Die Datenverarbeitungseinheit ist ausgebildet, Datensatz-Daten auszulesen und aus diesen Datensatz-Daten das zweite Anzeigesignal zu erzeugen und auszugeben. Dabei ist die Datenverarbeitungseinheit ausgebildet, das zweite Anzeigesignal derart zu erzeugen, dass eine durch einen Nutzer des Anzeigesystems über die Eingabeeinheit auszufüllende Anmeldemaske ausgeben wird, falls für aktuell ausgelesene Datensatz-Daten aus einer vorbestimmte Teilmenge von Datenarten nicht aktuell ein diesen Datenarten zugeordneter Berechtigungszustand des Anzeigesystems vorliegt.

Im Rahmen der Erfindung wurde erkannt, dass bei der Verwendung eines gegenüber dem elektromedizinischen Gerät separaten Anzeigesystems sichergestellt werden muss, dass die empfangenen Daten auf geeignete Weise visuell ausgegeben werden. Hierbei wurde weiterhin erkannt, dass im Klinikalltag mit personalisierten Daten gearbeitet wird, für die ein geeigneter Datenschutz sichergestellt werden sollte. Hierfür wird das Bereitstellen einer kombinierten visuellen Ausgabe vorgeschlagen, bei der sowohl Geräte-Daten eines elektromedizinischen Gerätes, wie beispielsweise physiologische Messwerte, als auch Datensatz-Daten, welche beispielsweise im Falle einer mangelnden Berechtigung eine auszufüllende Anmeldemaske indizieren, gemeinsam dargestellt werden.

Weiterhin wird vorteilhaft sichergestellt, dass das erfindungsgemäße Anzeigesystem unabhängig von elektromedizinischen Geräten betrieben wird.

An dem erfindungsgemäßen Anzeigesystem ist besonders vorteilhaft, dass empfangene Geräte-Daten, also beispielsweise physiologische Messwerte, angezeigt werden, während ausgelesene Datensatz-Daten durch eine Anmeldemaske gesperrt sind, falls aktuell nicht der für diese Daten erforderliche Berechtigungszustand vorliegt. Hierdurch wird ein schneller medizinischer Eingriff durch medizinisches Personal ermöglicht, auch wenn dieses nicht zur Einsicht der Datensatz-Daten berechtigt ist. Weiterhin wird im Notfall eine schnelle Behandlung ermöglicht, ohne dass vorher über die Anmeldemaske eine Veränderung des Berechtigungszustands erfolgen muss, auch wenn die behandelnde Person zur Einsicht in die Datensatz-Daten berechtigt wäre.

Beispielsweise handelt es sich bei den Datensatz-Daten, welche durch die Datenverarbeitungseinheit zum zweiten Anzeigesignal verarbeitet werden, um administrative Daten der Patientenbehandlung, wie etwa demographischen Daten des Patienten.

Ein weiterer Vorteil des erfindungsgemäßen Anzeigesystems besteht in der Klassifizierung der empfangenen Geräte-Daten in verschiedene Visualisierungstypen, sodass basierend auf den aktuell darzustellenden Visualisierungstypen eine jeweilige Darstellungsart dynamisch bestimmt werden kann. Die Darstellungsart umfasst typischerweise eine Darstellungsposition der auszugebenden Geräte-Daten, eine Darstellungsgröße dieser Geräte-Daten, eine Darstellungsschrift dieser Geräte-Daten und/oder eine Darstellungsfarbe der Geräte-Daten.

Die Geräte-Daten umfassen sämtliche Daten, die von dem mindestens einen elektromedizinischen Gerät empfangen werden und grenzen sich im Rahmen der Erfindung dadurch von den Datensatz-Daten ab, dass letztere durch die Datenverarbeitungseinheit ausgelesen und verarbeitet werden. Die Datensatz-Daten können beispielsweise von mindestens einem externen System empfangen werden, bei dem es sich nicht um ein elektromedizinisches Gerät handelt. Alternativ oder ergänzend können die Datensatz-Daten von einem Datensatz stammen, der durch ein Speichermodul der Datenverarbeitungseinheit bereitgestellt wird. Typischerweise handelt es sich bei dem mindestens einen externen System um ein Patienten-Management-System, das personenspezifische Daten als Datensatz-Daten ausgibt.

Das Auslesen der Datensatz-Daten kann sämtliche Arten einer Bereitstellung der Datensatz-Daten für die Datenverarbeitungseinheit umfassen. Insbesondere handelt es sich bei dem Empfang eines entsprechenden Signals und dem Auslesen der durch ein solches Signal empfangenen Daten um ein Auslesen im Sinne der vorliegenden Erfindung.

Das Vorsehen einer auszufüllenden Anmeldemaske ermöglicht vorteilhaft, dass lediglich Nutzer, die dazu berechtigt sind eine bestimmte personenspezifische Information wahrzunehmen, diese Information durch das Anzeigesystem sehen dürfen. Hierdurch ermöglicht das erfindungsgemäße Anzeigesystem ein besonders hohes Maß an Datenschutz.

Durch eine vorteilhafte Kompatibilität des erfindungsgemäßen Anzeigesystems mit verschiedenen elektromedizinischen Geräten, wird eine gleichzeitige Ausgabe von Geräte-Daten mehrerer elektromedizinischer Geräte ermöglicht. Weiterhin wird durch das erfindungsgemäße Anzeigesystem eine aufwändige Abstimmung zwischen Anzeigeeinheit und einem jeweiligen elektromedizinischen Gerät vermieden.

Die einzelnen Einheiten des erfindungsgemäßen Anzeigesystems können jeweils beabstandet voneinander oder innerhalb eines gemeinsamen Gehäuses angeordnet sein. Die Verarbeitung der Geräte-Daten kann dabei vorteilhaft durch einen einzigen Prozessor durchgeführt werden. Darüber hinaus kann es vorteilhaft sein, dass auch die Verarbeitung der weiteren Datensatz-Daten auf demselben Prozessor durchgeführt wird.

Das erfindungsgemäße Anzeigesystem kann beabstandet von dem mindestens einen elektromedizinischen Gerät angeordnet sein.

Das dynamische Bestimmen der jeweiligen Darstellungsart umfasst ein Bestimmen der Darstellungsart abhängig von den zugeordneten Visualisierungstypen entsprechend einer aktuell vorliegenden Anzahl an Visualisierungstypen. Somit erfolgt das dynamische Bestimmen nach dem Empfangen von Daten und einem entsprechenden Zuordnen eines Visualisierungstyps jeweils erneut. Dadurch liegt typischerweise kein fester Darstellungsort für einen bestimmten Visualisierungstyp vor, da dieser im Rahmen der dynamisch festgelegten Darstellungsart ebenfalls dynamisch festgelegt wird.

Die Gesamtheit aller Darstellungseigenschaften, wie beispielsweise Darstellungsposition, Darstellungsform, Darstellungsstil und/oder Darstellungsgröße, bildet die jeweilige Darstellungsart von empfangen Geräte-Daten.

Bei den empfangenen Geräte-Daten handelt es sich beispielsweise um physiologische Parameter, die durch das elektromedizinische Gerät bestimmt werden und/oder um eine Einstellung des elektromedizinischen Gerätes, wie etwa einen aktuellen Betriebsmodus, eine Alarmgrenze, eine Konfiguration und/oder ein aktuelles Patientenprofil, und/oder um einen Alarm, der durch das elektromedizinische Gerät bestimmt wurde.

Erfindungsgemäß kann das zweite Anzeigesignal und somit die Anzeige der Datensatz-Daten nicht durch die Anzeigesignaleinheit beeinflusst werden.

Nachfolgend werden bevorzugte Ausführungsformen des erfindungsgemäßen Anzeigesystems beschrieben.

Vorzugsweise indiziert mindestens eines der beiden Anzeigesignale eine Sequenz von einzelnen Bildern, einen Videodatenstrom und/oder einen Webseiten-Datenstrom.

Vorzugsweise werden das erste Anzeigesignal und/oder das zweite Anzeigesignal in vorbestimmten Zeitintervallen erzeugt, insbesondere periodisch erzeugt. Die vorbestimmten Zeitintervalle sind dabei so gewählt, dass sie einer typischen Dauer von klinisch relevanten Änderungen der von dem elektromedizinischen Gerät erzeugten Geräte-Daten entsprechen. Vorteilhaft betragen die vorbestimmten Zeitintervalle mindestens 0.1 Sekunden.

Vorzugsweise erfolgt die visuelle Ausgabe über ein Display der Anzeigeeinheit. In einer besonders bevorzugten Variante handelt es sich bei dem Display um ein Touchdisplay. In einer besonders bevorzugten Variante dieser Ausführungsform bildet das Touchdisplay zusätzlich die Benutzerschnittstelle der Eingabeeinheit, über die die Benutzereingabe erfolgt.

Vorzugsweise ist das Display umgeben von einem Gehäuse der Anzeigeeinheit.

Die Benutzereingabe kann beispielsweise durch die Eingabe einer Benutzeridentifikation, das Auslesen einer Schlüsselkarte und/oder das Auslesen von biometrischen Daten eines Nutzers realisiert sein. Entsprechende Realisierungen von Benutzerschnittstellen sind allgemein bekannt und werden daher im Folgenden nicht im Detail geschildert.

In einer besonders bevorzugten Ausführungsform weist das Anzeigesystem weiterhin eine Anzeigesignalüberwachungseinheit auf, die mit der Anzeigeeinheit und der Anzeigesignaleinheit signaltechnisch verbunden ist, und die ausgebildet ist, das erste Anzeigesignal von der Anzeigesignaleinheit zu empfangen und ein Ausgabesignal von der Anzeigeeinheit zu empfangen. Dabei enthält das Ausgabesignal Informationen zur erfolgten kombinierten visuelle Ausgabe. Die Anzeigesignalüberwachungseinheit ist weiterhin ausgebildet, erstes Anzeigesignal und Ausgabesignal basierend auf der jeweils bestimmten Darstellungsart miteinander zu vergleichen und im Falle einer mangelhaften Übereinstimmung ein Warnsignal auszugeben. In dieser Ausführungsform kann besonders vorteilhaft sichergestellt werden, dass die Ausgabe der Geräte-Daten und die Ausgabe der Datensatz-Daten derart erfolgt, dass eine Überlagerung von Daten vermieden wird und/oder besonders relevante Daten nicht von anderen Daten überlagert werden. Hierdurch können die Datensatz-Daten der Datenverarbeitungseinheit und die Geräte-Daten der elektromedizinischen Geräte in einer besonders gut aufeinander abgestimmten Art und Weise im Rahmen der kombinierten visuellen Ausgabe dargestellt werden. Besonders vorteilhaft erfolgt diese aufeinander abgestimmte kombinierte visuelle Ausgabe dadurch, dass durch das ausgegebene Warnsignal eine Veränderung der bestimmten Darstellungsart durch die Anzeigesignaleinheit ausgelöst wird. Das Vermeiden von Überlagerungen von Daten, wird in dieser Ausführungsform durch ein Anpassen der Ausgabe der Geräte-Daten ermöglicht. In einer alternativen und/oder ergänzenden Variante wird das Warnsignal direkt an einen Nutzer des Anzeigesystems ausgegeben. Eine solche Ausgabe an den Nutzer kann beispielsweise durch eine Darstellung einer Fehlermeldung über die Anzeigeeinheit realisiert sein.

Eine mangelnde Übereinstimmung wird von der Anzeigesignalüberwachungseinheit festgestellt, falls die erfolgte kombinierte visuelle Ausgabe ein vorbestimmtes Maß an Übereinstimmung mit den Geräte-Daten des ersten Anzeigesignals unterschreitet. So liegt beispielsweise eine mangelnde Übereinstimmung vor, falls Geräte-Daten aus einer vorbestimmten Teilmenge von besonders relevanten Datenarten nicht in der für den Nutzer erkennbaren kombinierten visuellen Ausgabe dargestellt werden. Dadurch wird vorteilhaft sichergestellt, dass besonders relevante Geräte-Daten nicht durch Datensatz-Daten, insbesondere administrative Daten, wie beispielsweise eine Anmeldemaske, eine Nutzerkennung, eine Patienteninformation und/oder eine Gerätekennung überdeckt werden. Das vorbestimmte Maß an Übereinstimmung kann sich ergänzend zum Inhalt der Geräte-Daten auch auf eine visuelle Ähnlichkeit beziehen, beispielsweise bei einem Vergleich der Ähnlichkeit von anzuzeigender Schriftart und angezeigter Schriftart, oder auf die Erkennbarkeit von dargestellten Geräte-Daten, beispielsweise durch eine Bewertung eines vorliegenden mangelnden Kontrastes zwischen einer Darstellungsfarbe und einer Hintergrundfarbe.

Eine mangelnde Übereinstimmung zwischen erfolgter kombinierter visueller Ausgabe und zu erfolgender Ausgabe der Geräte-Daten liegt in einer Ausführungsform dann vor, wenn relevante Geräte-Daten, beispielsweise klinische Daten, durch eine überlagerte Anzeige von weiteren Daten nicht oder nur teilweise im Rahmen der visuellen Ausgabe dargestellt werden. In einer weiteren Ausführungsform wird durch diesen Vergleich von erfolgter visueller Ausgabe und zu erfolgender visueller Ausgabe sichergestellt, dass dargestellte Geräte-Daten und insbesondere dargestellte Symbole von anderen Geräte-Daten, wie etwa einer anderen Ziffer, einem anderen Buchstaben oder einem anderen Symbol unterschieden werden können.

In einer Variante der oben beschriebenen Ausführungsformen ist die Anzeigesignalüberwachungseinheit weiterhin ausgebildet, zu prüfen, ob das Ausgabesignal Datensatz-Daten oder Geräte-Daten eines Visualisierungstyps aufweist, der ausschließlich bei Vorliegen eines bestimmten Berechtigungszustands ausgegeben werden darf und falls dieser Berechtigungszustand aktuell nicht vorliegt ein entsprechendes Warnsignal auszugeben. Hierdurch wird sichergestellt, dass ein Nutzer ohne entsprechende Berechtigung nicht geschützte Daten sehen kann. Diese Variante kombiniert vorteilhaft den Vorteil der Anzeigesignalüberwachungseinheit, dass die Daten des ersten Anzeigesignals und des zweiten Anzeigesignals derart kombiniert werden, dass durch eine Veränderung der Darstellungsart der Geräte-Daten eine aufeinander abgestimmte kombinierte visuelle Ausgabe möglich ist, mit dem Vorteil in der kombinierten visuellen Ausgabe nach geschützten Daten zu suchen. Vorzugsweise ist die Anzeigesignalüberwachungseinheit dafür dazu ausgebildet, durch das Ausgabesignal dargestellte Datensatz-Daten oder Geräte-Daten zu bestimmen und einem Visualisierungstyp zuzuordnen. Alternativ oder ergänzend ist die Anzeigesignalüberwachungseinheit dazu ausgebildet, den den entsprechenden in der kombinierten visuellen Ausgabe dargestellten Daten zugeordneten Visualisierungstyp anhand des Ausgabesignals auszulesen.

In einer weiteren vorteilhaften Ausführungsform ist die Datenverarbeitungseinheit weiterhin ausgebildet, ein von der Benutzereingabe abhängiges Berechtigungssignal zu empfangen und abhängig von dem Berechtigungssignal einen aktuell vorliegenden Berechtigungszustand des Anzeigesystems zu bestimmen, wobei für eine vorbestimmte Teilmenge von Datenarten eine Ausgabe der entsprechenden Datensatz-Daten über das zweite Anzeigesignal nur erfolgt, wenn aktuell ein diesen Datenarten zugeordneter Berechtigungszustand vorliegt. In dieser Ausführungsform erfolgt eine Ausgabe für die vorbestimmte Teilmenge von Datenarten an die Ausgabeeinheit durch das zweite Anzeigesignal nur bei Vorliegen eines entsprechenden Berechtigungszustands. Der aktuelle Berechtigungszustand wird vorteilhaft durch die Benutzereingabe festgelegt. Es gibt mindestens zwei verschiedene Berechtigungszustände, nämlich einen Grundzustand, der anzeigt, dass der Nutzer nicht berechtigt ist vertrauliche Daten zu sehen, und einen ersten Zustand, der anzeigt, dass der Nutzer berechtigt ist eine vorbestimmte Teilmenge von Datenarten, die vertrauliche Daten enthalten, zu sehen. Vorzugsweise wird zwischen weiteren Zuständen differenziert, sodass nur ein kleiner Personenkreis dazu berechtigt ist, sämtliche Datensatz-Daten zu sehen. Eine derartige Abstufung verschiedener Berechtigungszustände ermöglicht vorteilhaft eine Kontrolle darüber, welche Personen Zugang zu bestimmten vertraulichen Daten, wie beispielsweise personenbezogenen Daten haben. Hierdurch ermöglicht das erfindungsgemäße Anzeigesystem ein besonders hohes Maß an Schutz von personenbezogenen Daten, insbesondere unter Beibehaltung der Darstellung der Geräte-Daten.

In einer weiteren bevorzugten Ausführungsform umfasst die vorbestimmte Teilmenge von Datenarten personenbezogene Daten einer mit dem elektromedizinischen Gerät behandelten Person. Der Umgang mit derartigen Daten kann gesetzlichen Regelungen unterliegen, sodass ein Nachweis darüber, welcher Personenkreis Zugang zu welchen vertraulichen Daten hatte, vorteilhaft für die Einhaltung derartiger gesetzlicher Regelung sein kann. Weiterhin erlaubt das Anzeigesystem gemäß dieser Ausführungsform, behandlungsrelevante Geräte-Daten auszugeben, während personenspezifische Daten, die für die akute Behandlung der Person nicht relevant sind, der behandelnden Personen nicht als kombinierte visuelle Ausgabe ausgegeben werden. In einem weiteren Ausführungsbeispiel umfasst die vorbestimmte Teilmenge von Datenarten personenbezogene Daten der in der Vergangenheit mit dem elektromedizinischen Gerät behandelnden Ärzte. In einer weiteren ergänzenden oder alternativen Ausführungsform umfasst die vorbestimmte Teilmenge von Datenarten eine Behandlungshistorie der mit dem elektromedizinischen Gerät behandelten Person.

In einer weiteren Ausführungsformen des erfindungsgemäßen Anzeigesystems ist die Anzeigesignaleinheit weiterhin ausgebildet, das erste Anzeigesignal derart zu erzeugen, dass ein entsprechender Hinweis über die kombinierte visuelle Ausgabe bereitgestellt wird, falls aufgrund der Dimensionierung der kombinierten visuellen Ausgabe nicht alle von dem elektromedizinischen Gerät empfangenen Geräte-Daten durch die Anzeigeeinheit visuell ausgegeben werden können. Hierdurch wird ein Nutzer des erfindungsgemäßen Anzeigesystems darüber informiert, dass nicht alle empfangenen Daten über die Anzeigeeinheit dargestellt werden. In einer bevorzugten Variante dieser Ausführungsform können nicht dargestellte Geräte-Daten durch eine Interaktion zwischen Nutzer und Anzeigeeinheit dargestellt werden. Eine derartige Interaktion ist beispielsweise das Berühren einer entsprechenden Steuerfläche auf einem Touchdisplay der Anzeigeeinheit. In einem weiteren Beispiel dieser Variante ist eine derartige Interaktion das Nutzen einer Scroll-Leiste.

In einer weiteren bevorzugten Ausführungsform ist die Anzeigesignaleinheit weiterhin ausgebildet, eine Darstellungsgröße und/oder eine Darstellungsposition von empfangen Geräte-Daten eines Visualisierungstyps abhängig von einem diesen Visualisierungstyp zugeordneten Relevanzlevel zu bestimmen. In dieser Ausführungsform werden vorteilhaft besonders relevante Informationen anders dargestellt als weniger relevante Informationen. Insbesondere können besonders relevante Informationen durch eine größere Schriftgröße und/oder durch eine besonders zentrale Position innerhalb der kombinierten visuellen Ausgabe dargestellt werden. Durch die Verwendung verschiedener Darstellungsgrößen kann eine Anzahl an dargestellten Daten erhöht werden. Insbesondere können weniger relevante Informationen durch eine kleinere Schriftgröße dargestellt werden als relevante Informationen. Hierdurch kann eine große Anzahl von weniger relevanten Informationen gleichzeitig dargestellt werden, ohne dass dadurch die Darstellung von relevanten Informationen beeinträchtigt wird. In einer alternativen und/oder ergänzenden Ausführungsform umfasst die Darstellungsart als Darstellungseigenschaft neben der Darstellungsgröße und der Darstellungsposition einen Darstellungsstil. Der Darstellungsstil gibt beispielsweise eine zu nutzende Schriftart und/oder eine zu nutzende Schriftfarbe der darzustellenden Information an. Als Information ist hier und im Weiteren die jeweils durch empfangene Daten indizierte Information zu verstehen.

In einer besonders bevorzugten Ausführungsform des erfindungsgemäßen Anzeigesystems ist die Kommunikationsschnittstelle ausgebildet, Geräte-Daten von einer Mehrzahl von elektromedizinischen Geräten zu empfangen. Vorzugsweise umfasst dabei der den Geräte-Daten durch die Anzeigesignaleinheit zugeordnete Visualisierungstyp eine Geräteinformation, die das zu den Geräte-Daten korrespondierende elektromedizinische Gerät indiziert. In dieser Ausführungsform wird besonders vorteilhaft ausgenutzt, dass das erfindungsgemäße Anzeigesystem separat von dem elektromedizinischen Gerät funktioniert, dessen Geräte-Daten durch das Anzeigesystem bereitgestellt werden soll. In einer Variante dieser Ausführungsform umfasst der diesen Geräte-Daten zugeordnete Visualisierungstyp ein Relevanzlevel, das von dem zu diesen Geräte-Daten korrespondierenden elektromedizinischen Gerät abhängig ist.

Vorzugsweise empfängt die Anzeigesignaleinheit über die Kommunikationsschnittstelle sämtliche Geräte-Daten von elektromedizinischen Geräten innerhalb eines elektromedizinischen Kommunikationsnetzwerks. Ein solches elektromedizinisches Kommunikationsnetzwerk ist dem jeweiligen Anzeigesystem zugeordnet und umfasst typischerweise eine Mehrzahl von elektromedizinischen Geräten. In einem ersten Beispiel umfasst das elektromedizinische Kommunikationsnetzwerk sämtliche elektromedizinischen Geräte, die für die Behandlung eines einzigen konkreten Patienten verwendet werden. In einem zweiten Beispiel umfasst das elektromedizinische Kommunikationsnetzwerk sämtliche elektromedizinischen Geräte einer einzigen Geräteart, die auf einer Krankenhausstation verwendet werden.

Die Kommunikationsschnittstelle der erfindungsgemäßen Anzeigesignaleinheit erlaubt vorzugsweise eine kabellose Übertragung von Daten. In einer alternativen und/oder ergänzenden Ausführungsform erlaubt die Kommunikationsschnittstelle eine Kabel-basierte Übertragung von Daten. In einer weiteren Ausführungsform erlaubt die Kommunikationsschnittstelle sowohl eine kabellose Übertragung von Daten als auch eine Kabel-basierte Übertragung von Daten.

In einer weiteren Ausführungsform ist die Anzeigeeinheit beabstandet von der Anzeigesignaleinheit und/oder der Anzeigesignalüberwachungseinheit und/oder der Datenverarbeitungseinheit und/oder der Eingabeeinheit ausgebildet. In einer Variante dieser Ausführungsform sind die Anzeigesignaleinheit und die Anzeigesignalüberwachungseinheit in einem gemeinsamen Gehäuse angeordnet und mit der Anzeigeeinheit signaltechnisch verbunden. In einer weiteren Ausführungsform sind sämtliche Einheiten des erfindungsgemäßen Anzeigesystems in einem gemeinsamen Gehäuse abgeordnet.

In einer weiteren Ausführungsform umfasst das Anzeigesystem mindestens eine weitere Datenverarbeitungseinheit, die dazu ausgebildet ist, ein weiteres zweites Anzeigesignal auszugeben. Die Anzeigeeinheit ist hierbei vorzugsweise ausgebildet, die sich daraus ergebenden mindestens drei Anzeigesignale zu empfangen und basierend auf diesen Anzeigesignalen die kombinierte visuelle Ausgabe darzustellen.

Gemäß einem weiteren Aspekt der Erfindung wird die oben genannte Aufgabe gelöst durch ein Verfahren zum Anzeigen einer Ausgabe mindestens eines elektromedizinischen Gerätes.

Das erfindungsgemäße Verfahren weist die folgenden Schritte auf:
- Bereitstellen einer Benutzerschnittstelle, über die ein Nutzer eine Benutzereingabe bereitstellen kann;
- Empfangen von Geräte-Daten, die das elektromedizinische Gerät betreffen;
- Zuordnen der empfangenen Geräte-Daten zu einem jeweiligen Visualisierungstyp und dynamisches Bestimmen einer jeweiligen Darstellungsart der empfangenen Geräte-Daten basierend auf den aktuell darzustellenden Visualisierungstypen;
- Erzeugen und Ausgeben eines ersten Anzeigesignals basierend auf den empfangen Geräte-Daten und der jeweils bestimmten Darstellungsart;
- Auslesen von Datensatz-Daten;
- Erzeugen und Ausgeben eines zweiten Anzeigesignals basierend auf den Datensatz-Daten, wobei das zweite Anzeigesignal eine Darstellung einer auszufüllende Anmeldemaske indiziert, falls für aktuell ausgelesene Datensatz-Daten aus einer vorbestimmte Teilmenge von Datenarten nicht aktuell ein diesen Datenarten zugeordneter Berechtigungszustand vorliegt;
- Empfangen des ersten Anzeigesignals und des zweiten Anzeigesignals und Darstellen einer kombinierten visuellen Ausgabe basierend auf den beiden Anzeigesignalen.

Das Verfahren gemäß dem weiteren Aspekt der Erfindung erlaubt besonders vorteilhaft die Verwendung eines separaten Gerätes für das Anzeigen von Geräte-Daten eines elektromedizinischen Gerätes. Weiterhin wird vorteilhaft die Anzeige von Geräte-Daten verschiedener elektromedizinische Geräte durch ein einziges Anzeigemedium ermöglicht. Hierdurch kann eine besonders große Übersichtlichkeit der medizinischen Geräte-Daten sichergestellt werden. Weiterhin stellt das erfindungsgemäße Verfahren sicher, dass neben den empfangenen Geräte-Daten auch Datensatz-Daten, insbesondere nichtmedizinische Datensatz-Daten durch die Anzeigeeinheit darstellbar sind.

Schließlich erlaubt das erfindungsgemäße Verfahren vorteilhaft, dass Messwerte von elektromedizinischen Geräten angezeigt werden, während gleichzeitig ausgelesene, insbesondere vor dem Auslesen empfangene, personenspezifische Daten nicht ausgegeben werden und durch die auszufüllende Anmeldemaske gesperrt sind. Hierdurch kann im Notfall schnell eine medizinische Versorgung durch eine nicht zur Dateneinsicht berechtigten Person ermöglicht werden, oder ein schneller medizinischer Eingriff durch eine zur Dateneinsicht berechtigten Person ermöglicht werden, ohne dass diese Person eine Benutzereingabe bereitstellen muss.

Nachfolgend werden bevorzugte Ausführungsformen des erfindungsgemäßen Verfahrens beschrieben.

In einer besonders bevorzugten Ausführungsform umfasst das erfindungsgemäße Verfahren weiterhin die Schritte:
- Senden eines Ausgabesignals, das Informationen zur erfolgten kombinierten visuellen Ausgabe enthält;
- Empfangen des Ausgabesignals und des ersten Anzeigesignals und Vergleichen der beiden Signale miteinander;
- Ausgeben eines Warnsignals, falls eine mangelnde Übereinstimmung zwischen erfolgter kombinierter visueller Ausgabe und gemäß erstem Anzeigesignal zu erfolgender visueller Ausgabe vorliegt.
Diese Ausführungsform erlaubt vorteilhaft eine andauernde Überprüfung der Geräte-Daten in der kombinierten visuellen Ausgabe, sodass ein Fehler bei dieser Ausgabe schnell erkannt werden kann. Hierdurch ist das erfindungsgemäße Verfahren gemäß dieser Ausführungsform besonders wenig anfällig für Fehler bei der kombinierten visuellen Ausgabe. Insbesondere kann eine Überlappung von Geräte-Daten des ersten Anzeigesignals und von Datensatz-Daten des zweiten Anzeigesignals vermieden und/oder schnell korrigiert werden.

In einer weiteren Ausführungsform weist das erfindungsgemäße Verfahren die Schritte auf:
- Prüfen, ob das Ausgabesignal Geräte-Daten oder Datensatz-Daten eines Visualisierungstyps aufweist, der ausschließlich bei Vorliegen eines bestimmten Berechtigungszustands ausgegeben werden darf;
- Ausgeben eines entsprechenden Warnsignals falls dieser Berechtigungszustand aktuell nicht vorliegt.

In dieser Ausführungsform wird vorteilhaft sichergestellt, dass nur ein dazu befugter Personenkreis Zugang zu geschützten Daten hat.

In einer vorteilhaften Ausführungsform weist das erfindungsgemäße Verfahren weiterhin die Schritte auf:
- Empfangen eines von der Benutzereingabe abhängigen Berechtigungssignals;
- Bestimmen eines aktuell vorliegenden Berechtigungszustands basierend auf dem Berechtigungssignal, wobei für eine vorbestimmte Teilmenge von Datenarten eine Ausgabe der entsprechenden Datensatz-Daten über das zweite Anzeigesignal nur erfolgt, wenn aktuell ein diesen Datenarten zugeordneter Berechtigungszustand vorliegt.
In dieser Ausführungsform wird vorteilhaft bereits bei der Erzeugung des zweiten Anzeigesignals berücksichtigt, welche Datensatz-Daten angesichts des aktuell vorliegenden Berechtigungszustands ausgegeben werden dürfen.

In einer weiteren bevorzugten Ausführungsform ist das Verfahren zum Anzeigen einer Ausgabe einer Mehrzahl von elektromedizinischen Geräten ausgestaltet, mit dem zusätzlichen Schritt
- Empfangen von Geräte-Daten, die mindestens ein weiteres elektromedizinisches Gerät betreffen, wobei der den Geräte-Daten zugeordnete Visualisierungstyp eine Geräteinformation umfasst, die das zu den Daten korrespondierende elektromedizinische Gerät indiziert.
In dieser Ausführungsform wird vorteilhaft ausgenutzt, dass das erfindungsgemäße Verfahren unabhängig von dem jeweiligen elektromedizinischen Gerät ausgeführt werden kann. Hierdurch ist es insbesondere möglich, dass Geräte-Daten von der Mehrzahl von elektromedizinischen Geräten gleichzeitig durch das erfindungsgemäße Verfahren angezeigt werden können. Durch die jeweilige Geräteinformation lässt sich vorzugsweise auf ein Relevanzlevel oder eine Gruppe möglicher Relevanzlevel der empfangenen Daten schließen.

Weiterhin wird die oben genannte Aufgabe gelöst durch ein Programm mit einem Programmcode zur Durchführung des Verfahrens gemäß mindestens einer der oben angegebenen Ausführungsformen des erfindungsgemäßen Verfahrens, wenn der Programmcode auf einem Computer, einem Prozessor oder einer programmierbaren Hardwarekomponente ausgeführt wird.

Das Programm kann dabei auch nur einen Teil der erfindungsgemäßen Datenverarbeitung ausführen. Vorzugsweise werden zumindest die Funktion der Anzeigesignaleinheit, der Eingabeeinheit und der Datenverarbeitungseinheit durch ein Programm und/oder aufeinander abgestimmte Teil eines Programms gesteuert. Vorzugsweise wird das erfindungsgemäße Programm von einem Prozessor des Anzeigesystems ausgeführt. Alternativ wird das Programm zumindest durch einen ersten Prozessor der Anzeigesignaleinheit und durch einen zweiten Prozessor der Datenverarbeitungseinheit ausgeführt.

Die Erfindung soll nun anhand von in den Figuren schematisch dargestellten, vorteilhaften Ausführungsbeispielen näher erläutert werden. Von diesen zeigen im Einzelnen:
- Fig. 1: eine schematische Darstellung eines ersten Ausführungsbeispiels eines erfindungsgemäßen Anzeigesystems;
- Fig. 2: eine schematische Darstellung eines zweiten Ausführungsbeispiels des erfindungsgemäßen Anzeigesystems;
- Fig. 3: eine schematische Darstellung eines Dritten Ausführungsbeispiels des erfindungsgemäßen Anzeigesystems;
- Fig. 4: eine schematische Darstellung eines ersten Ausführungsbeispiels einer kombinierten visuellen Ausgabe des erfindungsgemäßen Anzeigesystems;
- Figs. 5, 6: eine schematische Darstellung der kombinierten visuellen Ausgabe des erfindungsgemäßen Anzeigesystems für die Anzeige einer nicht vollständigen Ausgabe von empfangenen Geräte-Daten (Fig. 5), und für ein verschobenes Fenster mit Datensatz-Daten (Fig.6);
- Fig. 7: ein Flussdiagramm eines ersten Ausführungsbeispiels eines erfindungsgemäßen Verfahrens.

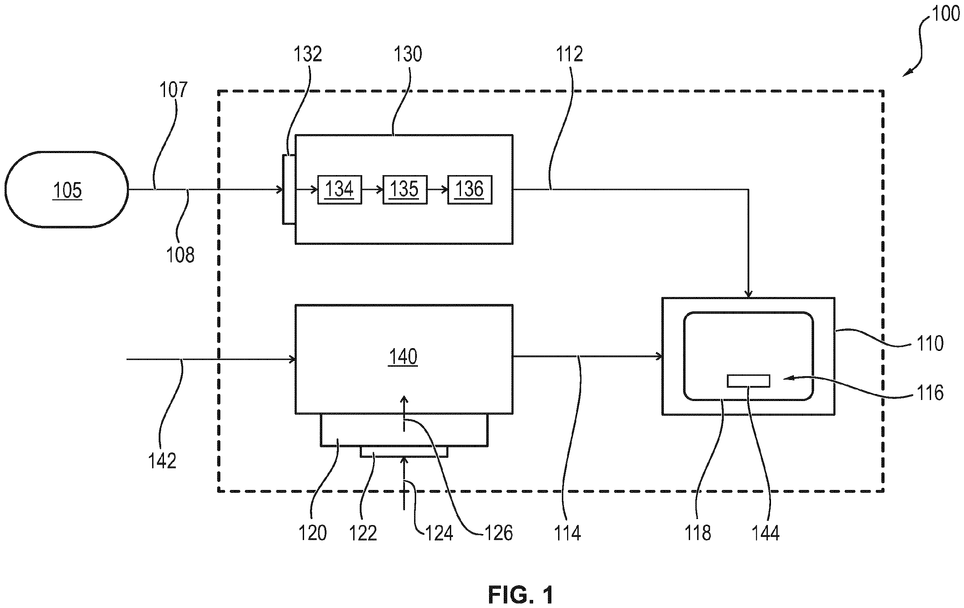

Fig. 1 zeigt eine schematische Darstellung eines ersten Ausführungsbeispiels eines erfindungsgemäßen Anzeigesystems 100.

Das erfindungsgemäße Anzeigesystem 100 zum Anzeigen einer Ausgabe mindestens eines elektromedizinischen Gerätes 105 umfasst eine Anzeigeeinheit 110, eine Eingabeeinheit 120, eine Anzeigesignaleinheit 130 und eine Datenverarbeitungseinheit 140.

Die Anzeigeeinheit 110 ist ausgebildet, ein erstes Anzeigesignal 112 und ein zweites Anzeigesignal 114 zu empfangen und basierend auf den beiden Anzeigesignalen 112, 114 eine kombinierte visuelle Ausgabe 116 darzustellen. Die kombinierte visuelle Ausgabe 116 erfolgt auf einem Display 118 der Anzeigeeinheit 110.

Die Eingabeeinheit 120 weist eine Benutzerschnittstelle 122 auf, über die ein Nutzer des Anzeigesystems 100 eine Benutzereingabe 124 bereitstellen kann. Bei der Benutzereingabe handelt es sich vorliegend um eine tastengebundene Eingabe einer über die Benutzerschnittstelle 122 bereitgestellten Identifikationsnummer. In einem nicht dargestellten Ausführungsbeispiel handelt es sich bei der Benutzereingabe um eine Eingabe durch das Auslesen biometrischer Daten. In einem weiteren nicht dargestellten Ausführungsbeispiel erfolgt die Benutzereingabe über das Auslesen einer entsprechenden Identifikationsinformation einer personengebundenen Schlüsselkarte. Vorliegend handelt es sich bei der Benutzerschnittstelle 122 um eine Tastatur. In einem nicht dargestellten Ausführungsbeispiel handelt es sich bei der Benutzerschnittstelle um ein Touch-Bedienelement, eine Computermaus oder um einen Joystick.

Die Anzeigesignaleinheit 130 weist eine Kommunikationsschnittstelle 132 zur Kommunikation mit dem mindestens einen elektromedizinischen Gerät 105 auf. Die Anzeigesignaleinheit 130 ist dabei dazu ausgebildet, Geräte-Daten 107, die das mindestens eine elektromedizinische Gerät 105 betreffen, über die Kommunikationsschnittstelle 132 zu empfangen. Hierfür empfängt die Kommunikationsschnittstelle 132 das die Geräte-Daten 107 indizierende Gerätesignal 108. Vorliegend erfolgt der Empfang des Gerätesignals 108 mit den Geräte-Daten 107 Kabel-basiert über eine Ethernet-Verbindung. In einem nicht dargestellten Ausführungsbeispiel erfolgt der Empfang des Gerätesignals über eine kabellose Verbindung, insbesondere über WLAN, Bluetooth, BLE oder ZigBee. In einem weiteren nicht dargestellten Ausführungsbeispiel wird die Kabel-basierte Verbindung durch ein Bussystem, wie etwa durch ein USB-System gebildet.

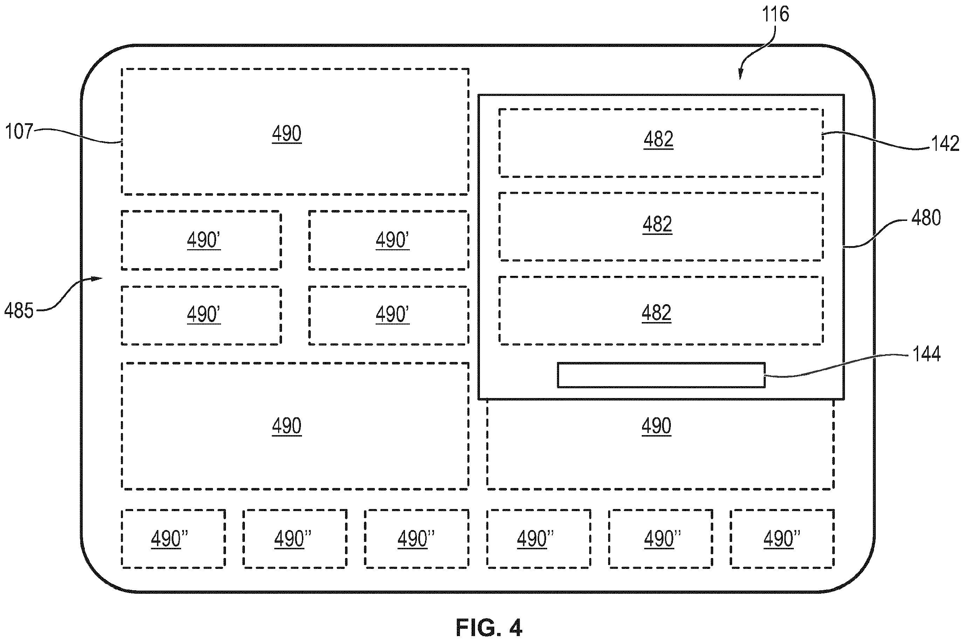

Im Rahmen eines ersten Verarbeitungsschrittes 134 ist die Anzeigesignaleinheit 130 ausgebildet, die Geräte-Daten 107 einem Visualisierungstypen zuzuordnen. In einem darauffolgenden zweiten Verarbeitungsschritt 135 wird abhängig von den aktuell darzustellenden Visualisierungstypen eine jeweilige Darstellungsart der empfangenen Geräte-Daten 107 dynamisch bestimmt. Basierend auf den Geräte-Daten 107 und der jeweils bestimmten Darstellungsart wird in einem darauf folgenden dritten Verarbeitungsschrittes 136 der Anzeigesignaleinheit 130 das erste Anzeigesignal 112 erzeugt und an die Anzeigeeinheit 110 ausgegeben. Vorliegend umfasst die bestimmte Darstellungsart von Geräte-Daten 107 als Darstellungseigenschaft eine Darstellungsposition der Geräte-Daten 107. Die Darstellungsposition beschreibt eine Position der Darstellung der entsprechenden Geräte-Daten 107 im Rahmen der kombinierten visuellen Ausgabe der Anzeigeeinheit 110. Beispiele für die Darstellungsposition und für die Darstellungseigenschaft sind im Rahmen der Figuren 4 bis 6 beschrieben.

Die Datenverarbeitungseinheit 140 ist ausgebildet, Datensatz-Daten 142 zu empfangen und aus diesen Datensatz-Daten 142 das zweite Anzeigesignal 114 zu erzeugen und auszugeben. Die Datensatz-Daten 142 sind beispielsweise personenbezogene Daten aus einem Patienten-Management-System. Insbesondere können die Datensatz-Daten 142 weiterhin Daten sein, die durch einen externen Computer (nicht dargestellt), beispielsweise einen zu Verwaltungszwecken verwendeten Computer, ausgegeben werden.

Die Datenverarbeitungseinheit 140 ist dabei dazu ausgebildet, das zweite Anzeigesignal 114 derart zu erzeugen, dass eine durch einen Nutzer des Anzeigesystems 100 über die Eingabeeinheit 120 auszufüllende Anmeldemaske 144 ausgegeben wird, falls für aktuell empfangene und dabei ausgelesene Datensatz-Daten 142 aus einer vorbestimmten Teilmenge von Datenarten nicht aktuell ein diesen Datenarten zugeordneter Berechtigungszustand des Anzeigesystems 100 vorliegt. Die Anmeldemaske 144 bildet ein Eingabefeld, in dem die Benutzereingabe 124, beispielsweise zum Eingeben einer vorbestimmten personenspezifischen Identifikationsnummer, erfolgen kann. Nach der Eingabe einer entsprechenden Identifikationsnummer würde die Datenverarbeitungseinheit 140 sämtliche Datensatz-Daten 142 ausgeben, die aktuell empfangen wurden, für die der Nutzer entsprechend seiner Identifikationsnummer eine Berechtigung besitzt. Hierdurch wird sichergestellt, dass ein Nutzer durch das erfindungsgemäße Anzeigesystem 100 nicht auf Daten zugreifen kann, für die er keine Berechtigung besitzt.

In dem dargestellten Ausführungsbeispiel umfasst die vorbestimmte Teilmenge von Datenarten personenbezogene Daten einer mit dem mindestens einen elektromedizinischen Gerät 105 behandelten Person.

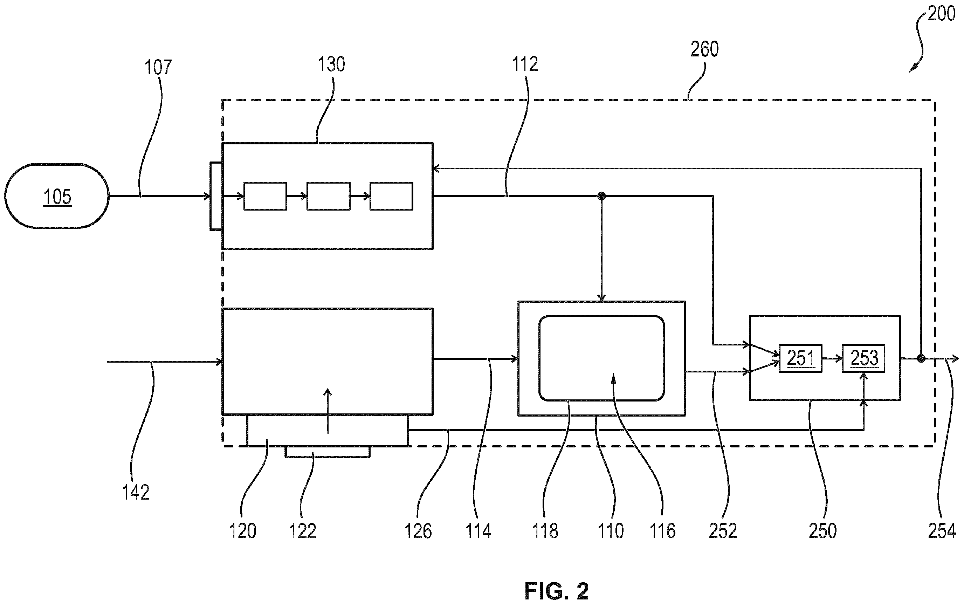

Der aktuell vorliegende Berechtigungszustand wird in dem dargestellten Ausführungsbeispiel durch die Datenverarbeitungseinheit 140 bestimmt. Hierbei wird ein von der Benutzereingabe 124 abhängiges Berechtigungssignal 126 von der Eingabeeinheit 120 ausgegeben und durch die Datenverarbeitungseinheit 140 empfangen. Abhängig von diesem Berechtigungssignal 126 wird daraufhin der aktuell vorliegende Berechtigungszustand des Anzeigesystems 100 bestimmt. Für die vorbestimmte Teilmenge von Datenarten erfolgt die Ausgabe der entsprechenden Datensatz-Daten 142 über das zweite Anzeigesignal 114 nur, wenn aktuell ein diesen Datenarten zugeordneter Berechtigungszustand vorliegt. In einem nicht dargestellten Ausführungsbeispiel sind sämtliche empfangene Datensatz-Daten in dem zweiten Anzeigesignal umfasst, und werden erst durch die Anzeigeeinheit 110 oder die im Rahmen von Fig. 2 erläuterte Anzeigesignalüberwachungseinheit aus der kombinierten visuellen Ausgabe 116 entfernt.

Der Berechtigungszustand wird vorliegend bestimmt durch einen Vergleich der über die Benutzerschnittstelle bereitgestellten Identifikationsnummer mit einer Gruppe von in einem Datenspeicher (nicht dargestellt) hinterlegten Identifikationsnummern, denen jeweils ein entsprechender Berechtigungszustand zugeordnet ist.

In einem nicht dargestellten Ausführungsbeispiel wird der Berechtigungszustand durch die Eingabeeinheit bestimmt und ein entsprechend angepasstes Berechtigungssignal ausgegeben, das den aktuell vorliegenden Berechtigungszustand indiziert.

Bei den empfangen Geräte-Daten 107 handelt es sich vorzugsweise um physiologische Parameter, die durch das mindestens eine elektromedizinische Gerät 105 bestimmt wurden und/oder um eine Einstellung des elektromedizinischen Gerätes 105 und/oder um einen Alarm, der durch das elektromedizinische Gerät 105 bestimmt wurde. Bei den elektromedizinischen Geräten 105 kann es sich beispielsweise um Patientenmonitore, Anästhesiesysteme, Beatmungssysteme, Wärmetherapiegeräte oder Infusionspumpen handeln. Bei den bestimmten physiologischen Parameter kann es sich beispielsweise um Messwerte handeln, wie etwa den invasiven oder nicht-invasiven Blutdruck, die Herzfrequenz, die Pulsrate, die Sauerstoffsättigung, die Atemfrequenz, die Körpertemperatur und/oder die Arrhythmie-Episoden. Bei den Einstellungen des elektromedizinischen Gerätes 105 kann es sich beispielsweise um eine Alarmgrenze, einen Betriebsmodus, einen erkannten Alarmzustand und/oder einen Signalisierungszustand handeln.

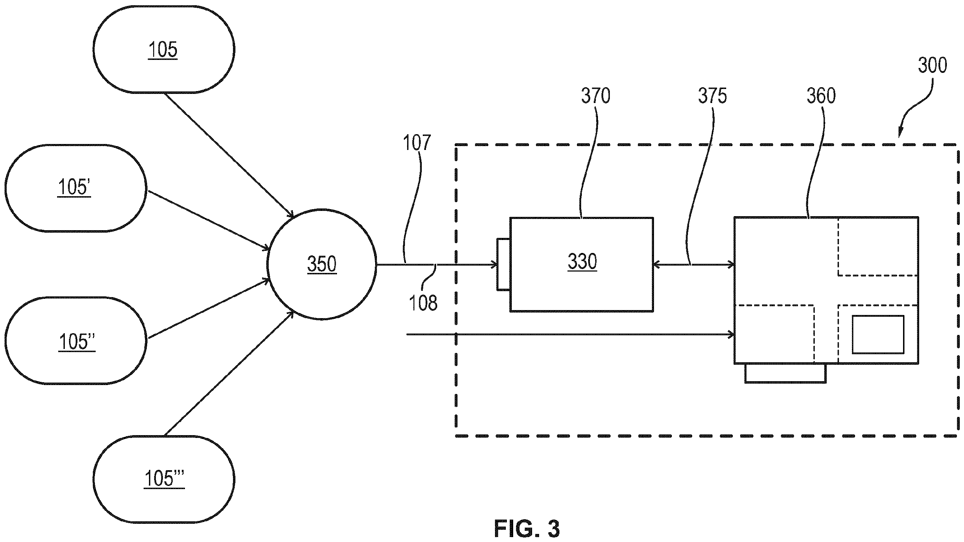

In dem dargestellten Ausführungsbeispiel sind sämtliche Einheiten 110, 120, 130 und 140 des Anzeigesystems 100 in einem gemeinsamen Gehäuse angeordnet. In anderen Ausführungsbeispielen sind diese Einheiten in getrennten Gehäusen angeordnet, wie im Rahmen von Fig. 3 dargestellt.

Fig. 2 zeigt eine schematische Darstellung eines zweiten Ausführungsbeispiels des erfindungsgemäßen Anzeigesystems 200.

Das Anzeigesystem 200 unterscheidet sich von dem in Fig. 1 dargestellten Anzeigesystem 100 dadurch, dass es zusätzlich eine Anzeigesignalüberwachungseinheit 250 aufweist.

Die Anzeigesignalüberwachungseinheit 250 ist mit der Anzeigeeinheit 110 und der Anzeigesignaleinheit 130 signaltechnisch verbunden. In dem dargestellten Ausführungsbeispiel handelt es sich dabei um eine Kabel-basierte Verbindung. Weiterhin ist die Anzeigesignalüberwachungseinheit 250 ausgebildet, das erste Anzeigesignal 112 von der Anzeigesignaleinheit 130 zu empfangen und ein Ausgabesignal 252 von der Anzeigeeinheit 110 zu empfangen. Das empfangene erste Anzeigesignal 112 kann sich von dem an die Anzeigeeinheit 110 ausgegebenen ersten Anzeigesignal 112 unterscheiden, insbesondere hinsichtlich Signaldauer und Ausgabehäufigkeit unterscheiden, wobei die beiden ersten Anzeigesignale 112 jedoch beide als gemeinsamen Signalanteile Informationen bezüglich der Darstellungsarten der empfangenen Geräte-Daten aufweisen. Das Ausgabesignal 252 indiziert dabei Informationen zur Darstellungsposition und zu weiteren Darstellungseigenschaften sämtlicher im Rahmen der erfolgten kombinierten visuelle Ausgabe 116 ausgegebenen Daten 107, 142. Weiterhin ist die Anzeigesignalüberwachungseinheit 250 ausgebildet, das empfangene erste Anzeigesignal 112 und das Ausgabesignal 252 in einem Vergleichsschritt 251 basierend auf der jeweils bestimmten Darstellungsart miteinander zu vergleichen und im Falle einer mangelhaften Übereinstimmung ein Warnsignal 254 auszugeben.

Das Warnsignal 254 wird vorliegend an einen Nutzer des Anzeigesystems 200 als optisches Signal ausgegeben (nicht dargestellt). Zusätzlich oder alternativ wird das Warnsignal 254 in diesem Ausführungsbeispiel an die Anzeigesignaleinheit 130 ausgegeben, hierdurch kann die Anzeigesignaleinheit 130 automatisiert eine Korrektur des ersten Anzeigesignals 112 durchführen.

Der Vergleich von erfolgter kombinierter visueller Ausgabe 116 und zu erfolgender visueller Ausgabe umfasst erfindungsgemäß auch eine Prüfung durch die Anzeigesignalüberwachungseinheit 250, ob alle darzustellenden Buchstaben, Ziffern und/oder Symbole für den Nutzer erkennbar sind. So wird erkannt, ob eine verwendete Schriftart für die Darstellung der Daten aktuell ungeeignet ist, ob Daten durch eine Ausgabe anderer Daten vollständig oder teilweise überdeckt werden und/oder ob eine Schriftfarbe für die Darstellung der Daten aktuell ungeeignet ist. Durch die Kombination von zweiten Anzeigesignal 114 und erstem Anzeigesignal 112 ist ein Überlappen bei der Darstellung von Daten und dabei insbesondere ein zumindest teilweises Überdecken von Geräte-Daten 107 durch Datensatz-Daten 142 möglich. Die Anzeigesignalüberwachungseinheit 250 ermöglicht mithin vorteilhaft sicherzustellen, dass keine besonders relevanten Geräte-Daten 107 durch die Datensatz-Daten 142 des zweiten Anzeigesignals 114 überdeckt werden.

Grundsätzlich bleibt erfindungsgemäß die Ausgabe der Datensatz-Daten 142 von der Anzeigesignalüberwachungseinheit 250 unbeeinflusst. Lediglich die Ausgabe der Geräte-Daten 107 kann durch das Warnsignal 254 derart beeinflusst werden, dass beispielsweise die Darstellungsposition, die Darstellungsgröße und/oder eine andere Darstellungseigenschaft der Geräte-Daten 107 derart beeinflusst wird, dass diese Geräte-Daten 107 für einen Nutzer des Anzeigesystems 200 deutlich erkennbar sind. Im Detail wird eine derartige Veränderung der Darstellungseigenschaft im Rahmen der Figuren 4 bis 6 dargestellt.

Weiterhin ist die Anzeigesignalüberwachungseinheit 250 in dem dargestellten Ausführungsbeispiel dazu ausgebildet, in einem Prüfschritt 253 zu prüfen, ob das Ausgabesignal 252 Datensatz-Daten 142 oder Geräte-Daten 107 eines Visualisierungstypen aufweist, der ausschließlich bei Vorliegen eines bestimmten Berechtigungszustands ausgegeben werden darf. Hierzu ist die Anzeigesignalüberwachungseinheit 250 dazu ausgebildet, anhand des Ausgabesignals 252 den Visualisierungstypen der ausgegebenen Datensatz-Daten 142 zu bestimmen. Weiterhin ist die Anzeigesignalüberwachungseinheit 252 dazu ausgebildet das von der Eingabeeinheit 120 ausgegebene Berechtigungssignal 126 zu empfangen und daraus den aktuell vorliegenden Berechtigungszustand zu bestimmen. Dadurch ist die erfindungsgemäße Anzeigesignalüberwachungseinheit 250 dazu ausgebildet, zu erkennen, ob aktuell ausgegebene Datensatz-Daten einem Visualisierungstypen zuzuordnen sind, der ausschließlich bei Vorliegen eines bestimmten Berechtigungszustands ausgegeben werden darf. Die Anzeigesignalüberwachungseinheit 250 ist weiterhin dazu ausgebildet, ein entsprechendes Warnsignal 254 auszugeben, falls dieser Berechtigungszustand aktuell nicht vorliegt.

In Fig. 2 ist weiterhin ein Gehäuse 260 der Anzeigeeinheit 200 dargestellt, das sowohl das Display 118 für die kombinierte visuelle Ausgabe 116, als auch die Benutzerschnittstelle 122 umgibt.

Fig. 3 zeigt eine schematische Darstellung eines dritten Ausführungsbeispiels des erfindungsgemäßen Anzeigesystems 300.

Das Anzeigesystem 300 unterscheidet sich von dem in Fig. 2 dargestellten Anzeigesystemen 200 dadurch, dass es dazu ausgebildet ist, die Ausgabe von vier verschiedenen elektromedizinischen Geräten 105, 105', 105", 105'" anzuzeigen. Die vier verschiedenen elektromedizinischen Geräte 105, 105', 105", 105'" sind ausgebildet, ihre Daten an ein Kommunikationsnetzwerk 350 auszugeben. Dies erfolgt vorliegend durch eine jeweilige kabellose Verbindung, beispielsweise über WLAN, Bluetooth, BLE oder ZigBee. In dem dargestellten Ausführungsbeispiel umfasst das Kommunikationsnetzwerk 350 sämtliche elektromedizinischen Geräte, die für die Behandlung eines einzigen Patienten genutzt werden. In einem alternativen nicht dargestellten Ausführungsbeispiel umfasst das Kommunikationsnetzwerk alle elektromedizinischen Geräte, die für die Messung eines bestimmten physiologischen Parameters oder Parametersatzes auf einer Krankenhausstation verwendet werden, um dadurch einen besonders guten Überblick über den entsprechenden physiologischen Zustand der Patienten dieser Station zu erhalten.

Die Anzeigesignaleinheit 330 ist hierbei dazu ausgebildet, das Gerätesignal 108, welches die Daten 107 umfasst, direkt von dem Kommunikationsnetzwerk 350 zu empfangen.

Weiterhin unterscheidet sich das Anzeigesystem 300 von den in Figs. 1 und 2 dargestellten Anzeigesystemen 100, 200 dadurch, dass die Anzeigesignaleinheit 330 in einem separaten Gehäuse 370 angeordnet ist, das beabstandet von einem gemeinsamen Gehäuse 360 der übrigen Einheiten des Anzeigesystems 300 angeordnet ist. In dem dargestellten Ausführungsbeispiel ermöglicht eine bidirektionale Funkverbindung 375 zwischen Anzeigesignaleinheit 330 und einem zentralen Prozessor (nicht dargestellt) innerhalb des gemeinsamen Gehäuses 360 eine erfindungsgemäße Kommunikation zwischen den Einheiten. In einem nicht dargestellten Ausführungsbeispiel ermöglicht eine Kabel-basierte bidirektionale Verbindung eine Kommunikation zwischen den Einheiten. In einem weiteren nicht dargestellten Ausführungsbeispiel kommuniziert die Anzeigesignaleinheit über eine unibidirektionale Verbindung mit der Anzeigeeinheit.

Fig. 4 zeigt eine schematische Darstellung eines ersten Ausführungsbeispiels einer kombinierten visuellen Ausgabe 116 des erfindungsgemäßen Anzeigesystems 200.

Die kombinierte visuelle Ausgabe 116 kombiniert dabei eine administrative Ausgabe 480 der Datensatz-Daten 142 und der Anmeldemaske 144 entsprechend dem zweiten Anzeigesignal 114 mit der medizinischen Ausgabe 485 der Geräte-Daten 107 entsprechend dem ersten Anzeigesignal. Die medizinische Ausgabe 485 umfasst vorliegend den gesamten Teil der kombinierten visuellen Ausgabe 116, der nicht zu der administrativen Ausgabe 480 gehört.

Die kombinierte visuelle Ausgabe 116 verfügt über eine Vielzahl von gestrichelt dargestellten Ausgabebereichen 490, 490', 490". Die konkreten Datenspezifischen Inhalte der Ausgabebereiche 490, 490', 490" wurden aus Gründen der Übersichtlichkeit nicht dargestellt. Die Darstellung von gestrichelten oder geschlossenen Rahmen, die im Folgenden verwendet werden, dient lediglich der besseren Visualisierung der Ausgabebereiche 490, 490', 490", wobei vorzugsweise für einen Nutzer des Anzeigesystems 200 keine Rahmen innerhalb der kombinierten visuellen Ausgabe 116 erkennbar sind. Die Ausgabebereiche unterscheiden sich dabei in ihrer Größe. In dem dargestellten Ausführungsbeispiel hat die Anzeigeeinheit drei Geräte-Daten empfangen, die einem Visualisierungstypen zugeordnet wurden, dem ein hohes Relevanzlevel zugeordnet ist. Diese drei Ausgaben entsprechen den drei großen Ausgabebereichen 490. Ein Beispiel für einen solchen Visualisierungstyp mit hohem Relevanzlevel ist ein physiologischer Messwert, wie beispielsweise die Sauerstoffsättigung im Blut. Weiterhin wurden Geräte-Daten mit einem mittleren Relevanzlevel empfangen, denen vier Ausgabebereiche 490' mittlerer Größe zugeordnet wurden. Letztlich wurden auch Geräte-Daten mit einem geringen Relevanzlevel enthalten, denen sechs Ausgabebereiche 490" kleiner Größe durch die Anzeigesignaleinheit zugeordnet wurden.

Die großen Ausgabebereiche 490 sind sowohl im oberen Bereich, als auch im zentralen Bereich der kombinierten visuellen Ausgabe 116 angeordnet und dadurch auch aufgrund ihrer Position für einen Nutzer des Anzeigesystems 200 schnell zu erkennen.

Die Anordnung der Ausgabebereiche und die Auswahl von deren Größe wurde von der Anzeigesignaleinheit aufgrund der aktuell darzustellenden Visualisierungstypen dynamisch bestimmt. Für den Fall, dass aktuell andere Visualisierungstypen darzustellen wären, würde mithin das gleiche Anzeigesystem eine andere Anordnung von Ausgabebereichen vorsehen.

Die Darstellungsgröße und die Darstellungsposition von empfangenen Daten bilden in diesem Ausführungsbeispiel Darstellungseigenschaften. Die Gesamtheit aller Darstellungseigenschaften bildet die jeweilige Darstellungsart von empfangenen Daten. Nicht dargestellt ist in Fig. 4, dass eine Schriftart und eine Schriftfarbe der darzustellenden Daten ebenfalls eine Darstellungseigenschaft bilden.

In einem nicht dargestellten erfindungsgemäßen Ausführungsbeispiel bilden lediglich die Darstellungsposition und eine Information, ob eine Darstellung der Daten erfolgt, eine jeweilige Darstellungseigenschaft der dynamisch bestimmten Darstellungsart.

Die administrative Ausgabe 480 umfasst drei gleichgroße administrative Ausgabebereiche 482 und die Anmeldemaske 144. In den administrativen Ausgabebereichen 482 werden die Datensatz-Daten 142 dargestellt. Die Anzeigesignaleinheit und die Anzeigesignalüberwachungseinheit haben in dem dargestellten Ausführungsbeispiel keinen Einfluss auf die administrative Ausgabe 480. In einem nicht dargestellten Ausführungsbeispiel ist die Anzeigesignalüberwachungseinheit dazu ausgebildet, eine die administrative Ausgabe überlagernde Fehlerausgabe über die Anzeigesignaleinheit auszulösen, falls die dargestellten Datensatz-Daten angesichts des aktuell vorliegenden Berechtigungszustands nicht ausgegeben werden dürfen.

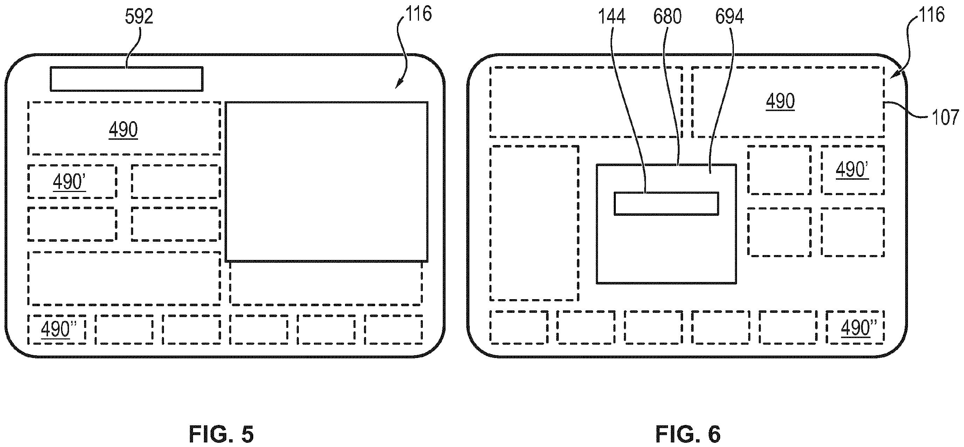

Die Anzeigesignalüberwachungseinheit hat in dem dargestellten Ausführungsbeispiel der kombinierten visuellen Ausgabe 116 erkannt, dass, trotz der administrativen Ausgabe 480 und einer geringfügigen Überlappung mit einem Ausgabebereich 490 mit hohem zugeordneten Relevanzlevel, sämtliche Geräte-Daten 107 für den Nutzer des Anzeigesystems erkennbar sind und mithin keine Veränderung des ersten Anzeigesignals und auch keine Ausgabe eines Warnsignals erforderlich ist. Der Fall einer Veränderung der Darstellungsposition durch die Anzeigesignalüberwachungseinheit aufgrund einer Überlappung ist in Fig. 6 beschrieben.

Figs. 5 und 6 zeigen eine schematische Darstellung der kombinierten visuellen Ausgabe 116 des erfindungsgemäßen Anzeigesystems für die Anzeige einer nicht vollständigen Ausgabe 592 von empfangenen Daten (Fig. 5), und für ein verschobenes Fenster 694 mit Datensatz-Daten, bei denen es sich nicht um Geräte-Daten handelt (Fig.6).

Die in Fig. 5 und 6 dargestellten kombinierten visuellen Ausgaben 116 gehen von der in Fig. 4 gezeigten kombinierten visuellen Ausgabe 116 aus, und zeigen, wie diese kombinierte visuelle Ausgabe 116 durch die Anzeige einer nicht vollständig Ausgabe 592 und durch eine Veränderung der administrativen Ausgabe 680 verändert wird.

Die Anzeige einer nicht vollständigen Ausgabe 592 erfolgt typischerweise in einem Randbereich der visuellen Ausgabe 116, vorliegend am oberen Rand. Die Anzeige einer nicht vollständigen Ausgabe 592 informiert einen Nutzer des Anzeigesystems darüber, dass aufgrund der Dimensionierung der visuellen Ausgabe nicht alle von dem Anzeigesystem empfangenen Geräte-Daten durch die Anzeigeeinheit visuell ausgegeben werden können. Diese Anzeige führt ebenfalls dazu, dass die anderen Ausgabebereiche 490, 490', 490" verschoben und verkleinert werden. Vorliegend erfolgt die Verkleinerung der Ausgabebereiche 490, 490', 490" derart, dass die Größenverhältnisse der Ausgabebereich 490, 490', 490" und der administrativen Ausgabe 480 untereinander gleich geblieben sind.

Fig. 6 zeigt eine gegenüber Fig. 4 dahingehend veränderte kombinierte visuelle Ausgabe 116, dass die administrative Ausgabe 680 durch das zweite Anzeigesignal eine andere Darstellungsposition einnimmt, als in Fig. 4. In dem in Fig. 1 dargestellten Ausführungsbeispiel des Anzeigesystems 100 führt eine derartige Verschiebung der administrativen Ausgabe 680 zu einer Überlagerung dieser Ausgabe mit den entsprechenden Ausgabebereichen 490, 490', 490" für die Geräte-Daten 107. Für das Anzeigesystem 100 ist daher vorzugsweise ein bestimmter Bereich der kombinierten visuellen Ausgabe 116 für die administrative Ausgabe 680 vorgesehen, sodass eine Überlagerung nur bei einer unvorhergesehenen Verschiebung der administrativen Ausgabe 680 erfolgt. Für das dargestellte Ausführungsbeispiel des Anzeigesystems 200 ermöglicht die Überprüfung des Ausgabesignals durch die Anzeigesignalüberwachungseinheit eine Korrektur der Darstellungsposition der großen und mittleren Ausgabebereiche 490, 490' derart, dass eine Überlagerung vermieden wird. Hierbei wird auch die Darstellungsgröße dieser Ausgabebereiche 490, 490' derart verändert, dass es keine Überschneidung zwischen den Ausgabebereich und der administrative Ausgabe 680 gibt.

In dem in Fig. 6 dargestellten Ausführungsbeispiel erkennt die Datenverarbeitungseinheit, dass für aktuell empfangene und ausgelesene Datensatz-Daten aus einer vorbestimmten Teilmenge von Datenarten nicht aktuell ein diesen Datenarten zugeordneter Berechtigungszustand des Anzeigesystems 200 vorliegt, sodass eine auszufüllende Anmeldemaske 144 anstelle dieser Daten ausgegeben wird.

Die dargestellten Ausgabebereiche 490, 490', 490" sind nicht einschränkend zu verstehen. In nicht dargestellten Ausführungsbeispielen sind andere Größen, Positionen und Formen von Ausgabebereichen vorgesehen. Die Daten können dabei innerhalb der Ausgabenbereiche in Form von Buchstaben, Ziffern, Symbolen, Tabellen, Diagrammen und von jeglicher weiteren graphischen Darstellung ausgegeben werden.

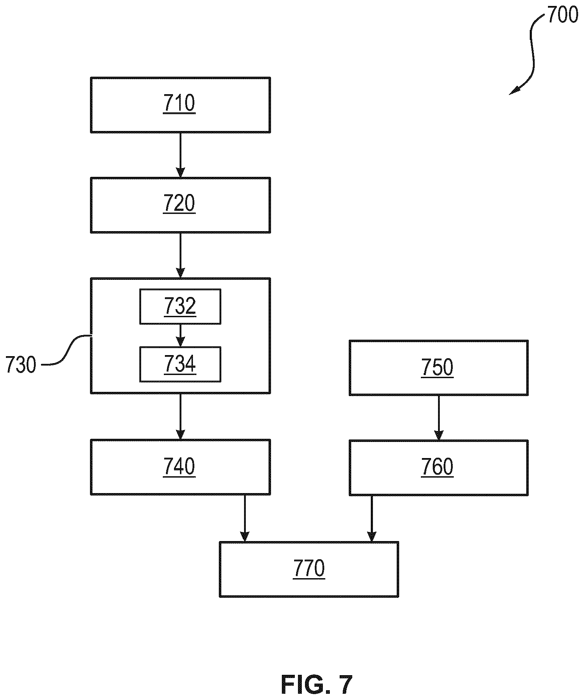

Fig. 7 zeigt ein Flussdiagramm eines ersten Ausführungsbeispiels eines erfindungsgemäßen Verfahrens 700.

Das erfindungsgemäße Verfahren 700 zum Anzeigen einer Ausgabe eines elektromedizinischen Gerätes umfasst eine Abfolge der folgenden beschriebenen Schritte:
Ein erster Schritt 710 umfasst ein Bereitstellen einer Benutzerschnittstelle, über die ein Nutzer eine Benutzereingabe bereitstellen kann.

Ein weiterer Schritt 720 umfasst ein Empfangen von Geräte-Daten, die das elektromedizinische Gerät betreffen.

Ein nächster Schritt 730 umfasst in einem ersten Teilschritt 732 ein Zuordnen der empfangenen Geräte-Daten zu einem jeweiligen Visualisierungstyp und in einem zweiten Teilschritt 734 ein dynamisches Bestimmen einer jeweiligen Darstellungsart der empfangenen Geräte-Daten basierend auf den aktuell darzustellenden Visualisierungstypen.

Ein darauffolgender Schritt 740 umfasst ein Erzeugen und Ausgeben eines ersten Anzeigesignals basierend auf den empfangen Geräte-Daten und der jeweils bestimmten Darstellungsart.

Ein von den vorhergehenden Schritten unabhängiger Schritt 750 umfasst ein Auslesen von Datensatz-Daten.

Ein darauffolgender Schritte 760 umfasst ein Erzeugen und Ausgeben eines zweiten Anzeigesignals basierend auf den Datensatz-Daten, wobei das zweite Anzeigesignal eine Darstellung einer auszufüllenden Anmeldemaske indiziert, falls für aktuell ausgelesene Datensatz-Daten aus einer vorbestimmte Teilmenge von Datenarten nicht aktuell ein diesen Datenarten zugeordneter Berechtigungszustand vorliegt.

Ein abschließender Schritt 770 umfasst ein Empfangen des ersten Anzeigesignals und des zweiten Anzeigesignals und ein Darstellen einer kombinierten visuellen Ausgabe basierend auf den beiden Anzeigesignalen.

Der Schritt 710 wird typischerweise bei der Herstellung der entsprechenden Vorrichtung einmalig ausgeführt.

Die Schritte 720 bis 740 erfolgen vorzugsweise aufeinanderfolgend, wobei das empfangen von Geräte-Daten entsprechend Schritt 720 die weiteren Schritte auslöst. Die aufeinanderfolgenden Schritte 750 und 760 erfolgen vorzugsweise unabhängig von den Schritten 720 bis 740 durch den Empfang der Datensatz-Daten.

Die Schritte 720 bis 740 und 770 erfolgen vorzugsweise in regelmäßigen zeitlichen Abständen, die so gewählt sind, dass die kombinierte visuelle Ausgabe nahezu in Echtzeit mit dem Empfang der Daten erfolgt.

Falls keine Datensatz-Daten ausgelesen wurden, wird dennoch ein zweites Anzeigesignal ausgelöst, welches die Information eines nicht erfolgten Bereitstellens von Datensatz-Daten indiziert. In einem nicht dargestellten Ausführungsbeispiel erfolgt keine Ausgabe eines zweiten Anzeigesignals, falls keine Datensatz-Daten empfangen wurden. Entsprechend wird bei der Darstellung der kombinierten visuellen Ausgabe lediglich das erste Anzeigesignal berücksichtigt.

### Bezugszeichenliste

- 100, 200, 300: Anzeigesystem
- 105, 105', 105", 105'": elektromedizinisches Gerät
- 107: Gerät-Daten
- 108: Gerätesignal
- 110: Anzeigeeinheit
- 112: erstes Anzeigesignal
- 114: zweites Anzeigesignal
- 116: kombinierte visuelle Ausgabe
- 118: Display
- 120: Eingabeeinheit
- 122: Benutzerschnittstelle
- 124: Benutzereingabe
- 126: Berechtigungssignal
- 130, 330: Anzeigesignaleinheit
- 132: Kommunikationsschnittstelle
- 134: erster Verarbeitungsschritt
- 135: zweiter Verarbeitungsschritt
- 136: dritter Verarbeitungsschritt
- 140: Datenverarbeitungseinheit
- 142: Datensatz-Daten
- 144: Anmeldemaske
- 250: Anzeigesignalüberwachungseinheit
- 251: Vergleichsschritt
- 252: Ausgabesignal
- 253: Prüfschritt
- 254: Warnsignal
- 260, 360: Gehäuse
- 350: Kommunikationsnetzwerk
- 370: separates Gehäuse
- 375: bidirektionale Funkverbindung
- 480, 680: administrative Ausgabe
- 482: administrativer Ausgabebereich
- 485: medizinische Ausgabe
- 490, 490', 490": Ausgabebereich
- 592: Anzeige einer nicht vollständigen Ausgabe
- 694: verschobenes Fenster
- 700: Verfahren
- 710, 720, 730, 740, 750, 760, 770: Verfahrensschritte
- 732, 734: Teilschritte des Verfahrensschrittes 730

## Patentansprüche

1. Anzeigesystem (100) zum Anzeigen einer Ausgabe mindestens eines elektromedizinischen Gerätes (105), mit
- einer Anzeigeeinheit (110), die ausgebildet ist, ein erstes Anzeigesignal (112) und mindestens ein zweites Anzeigesignal (114) zu empfangen und basierend auf den beiden Anzeigesignalen (112, 114) eine kombinierte visuelle Ausgabe (116) darzustellen,
- einer Eingabeeinheit (120), die eine Benutzerschnittstelle (122) aufweist, über die ein Nutzer eine Benutzereingabe (124) bereitstellen kann,
- einer Anzeigesignaleinheit (130), die eine Kommunikationsschnittstelle (132) zur Kommunikation mit dem mindestens einen elektromedizinischen Gerät (105) aufweist und ausgebildet ist, Geräte-Daten (107), die das elektromedizinische Gerät (105) betreffen, über die Kommunikationsschnittstelle (132) zu empfangen, diese Geräte-Daten (107) einem Visualisierungstyp zuzuordnen und abhängig von den aktuell darzustellenden Visualisierungstypen eine jeweilige Darstellungsart der empfangenen Geräte-Daten (107) dynamisch zu bestimmen und basierend auf den Geräte-Daten (107) und der jeweils bestimmten Darstellungsart das erste Anzeigesignal (112) zu erzeugen und an die Anzeigeeinheit (110) auszugeben,
- einer Datenverarbeitungseinheit (140), die ausgebildet ist, Datensatz-Daten (142) auszulesen und aus diesen Datensatz-Daten (142) das zweite Anzeigesignal (114) zu erzeugen und auszugeben, und wobei die Datenverarbeitungseinheit (140) weiter ausgebildet ist, das zweite Anzeigesignal (114) derart zu erzeugen, dass eine durch einen Nutzer des Anzeigesystems (100) über die Eingabeeinheit (120) auszufüllende Anmeldemaske (144) ausgeben wird, falls für aktuell ausgelesene Datensatz-Daten (142) aus einer vorbestimmte Teilmenge von Datenarten nicht aktuell ein diesen Datenarten zugeordneter Berechtigungszustand des Anzeigesystems (100) vorliegt.

2. Anzeigesystem (200) gemäß Anspruch 1, wobei das Anzeigesystem (200) weiterhin eine Anzeigesignalüberwachungseinheit (250) aufweist, die mit der Anzeigeeinheit (110) und der Anzeigesignaleinheit (130) signaltechnisch verbunden ist, und die ausgebildet ist, das erste Anzeigesignal (112) von der Anzeigesignaleinheit (130) zu empfangen und ein Ausgabesignal (252) von der Anzeigeeinheit (110) zu empfangen, wobei das Ausgabesignal (252) Informationen zur erfolgten kombinierten visuelle Ausgabe (116) enthält, und wobei die Anzeigesignalüberwachungseinheit (250) ausgebildet ist, erstes Anzeigesignal (112) und Ausgabesignal (252) basierend auf der jeweils bestimmten Darstellungsart miteinander zu vergleichen und im Falle einer mangelhaften Übereinstimmung ein Warnsignal (254) auszugeben.

3. Anzeigesystem (200) gemäß Anspruch 2, wobei die Anzeigesignalüberwachungseinheit (250) weiterhin ausgebildet ist, zu prüfen, ob das Ausgabesignal (252) Geräte-Daten (107) oder Datensatz-Daten (142) eines Visualisierungstyps aufweist, der ausschließlich bei Vorliegen eines bestimmten Berechtigungszustands ausgegeben werden darf und falls dieser Berechtigungszustand aktuell nicht vorliegt ein entsprechendes Warnsignal (154) auszugeben.

4. Anzeigesystem (100, 200) gemäß mindestens einem der vorhergehenden Ansprüche, wobei die Datenverarbeitungseinheit (140) weiterhin ausgebildet ist, ein von der Benutzereingabe (124) abhängiges Berechtigungssignal (126) zu empfangen und abhängig von dem Berechtigungssignal (126) einen aktuell vorliegenden Berechtigungszustand des Anzeigesystems (100, 200) zu bestimmen, wobei für eine vorbestimmte Teilmenge von Datenarten eine Ausgabe der entsprechenden Datensatz-Daten (142) über das zweite Anzeigesignal (114) nur erfolgt, wenn aktuell ein diesen Datenarten zugeordneter Berechtigungszustand vorliegt.

5. Anzeigesystem (100) gemäß mindestens einem der vorhergehenden Ansprüche, wobei die vorbestimmte Teilmenge von Datenarten personenbezogene Daten einer mit dem elektromedizinischen Gerät (105) behandelten Person umfasst.

6. Anzeigesystem (100) gemäß mindestens einem der vorhergehenden Ansprüche, wobei die Anzeigesignaleinheit (130) weiterhin ausgebildet ist, das erste Anzeigesignal (112) derart zu erzeugen, dass ein entsprechender Hinweis über die kombinierte visuelle Ausgabe (116) bereitgestellt wird, falls aufgrund der Dimensionierung der kombinierten visuellen Ausgabe (116) nicht alle von dem elektromedizinischen Gerät (105) empfangenen Geräte-Daten (107) durch die Anzeigeeinheit (110) visuell ausgegeben werden können.

7. Anzeigesystem (100) gemäß mindestens einem der vorhergehenden Ansprüche, wobei Anzeigesignaleinheit (130) weiterhin ausgebildet ist, eine Darstellungsgröße und/oder eine Darstellungsposition von empfangen Geräte-Daten (107) eines Visualisierungstyps abhängig von einem diesen Visualisierungstyp zugeordneten Relevanzlevel zu bestimmen.

8. Anzeigesystem (300) gemäß mindestens einem der vorhergehenden Ansprüche, wobei die Kommunikationsschnittstelle (132) ausgebildet ist, Geräte-Daten (107) von einer Mehrzahl von elektromedizinischen Geräten (105, 105', 105", 105''') zu empfangen, wobei der den Geräte-Daten (107) durch die Anzeigesignaleinheit (130) zugeordnete Visualisierungstyp eine Geräteinformation umfasst, die das zu den Geräte-Daten (107) korrespondierende elektromedizinische Gerät (105, 105', 105", 105'") indiziert.

9. Verfahren (700) zum Anzeigen einer Ausgabe mindestens eines elektromedizinischen Gerätes (105), aufweisend die folgenden Schritte
- Bereitstellen einer Benutzerschnittstelle (122), über die ein Nutzer eine Benutzereingabe (124) bereitstellen kann;
- Empfangen von Geräte-Daten (107), die das mindestens eine elektromedizinische Gerät (105) betreffen;
- Zuordnen der empfangenen Geräte-Daten (107) zu einem jeweiligen Visualisierungstyp und dynamisches Bestimmen einer jeweiligen Darstellungsart der empfangenen Geräte-Daten (107) basierend auf den aktuell darzustellenden Visualisierungstypen;
- Erzeugen und Ausgeben eines ersten Anzeigesignals (112) basierend auf den empfangen Geräte-Daten (107) und der jeweils bestimmten Darstellungsart;
- Auslesen von Datensatz-Daten (142);
- Erzeugen und Ausgeben eines zweiten Anzeigesignals (114) basierend auf den Datensatz-Daten (142), wobei das zweite Anzeigesignal (114) eine Darstellung einer auszufüllende Anmeldemaske (144) indiziert, falls für aktuell ausgelesene Datensatz-Daten (142) aus einer vorbestimmte Teilmenge von Datenarten nicht aktuell ein diesen Datenarten zugeordneter Berechtigungszustand vorliegt;
- Empfangen des ersten Anzeigesignals (112) und des zweiten Anzeigesignals (114) und Darstellen einer kombinierten visuellen Ausgabe (116) basierend auf den beiden Anzeigesignalen (112, 114).

10. Verfahren gemäß Anspruch 9, weiterhin aufweisend die Schritte
- Senden eines Ausgabesignals (252), das Informationen zur erfolgten kombinierten visuellen Ausgabe (116) enthält;
- Empfangen des Ausgabesignals (252) und des ersten Anzeigesignals (112) und Vergleichen der beiden Signale miteinander;
- Ausgeben eines Warnsignals (254), falls eine mangelnde Übereinstimmung zwischen erfolgter kombinierter visueller Ausgabe (116) und gemäß erstem Anzeigesignal (112) zu erfolgender visueller Ausgabe vorliegt.

11. Verfahren gemäß Anspruch 10, weiterhin aufweisend die Schritte
- Prüfen, ob das Ausgabesignal (252) Geräte-Daten (107) oder Datensatz-Daten (107) eines Visualisierungstyps aufweist, der ausschließlich bei Vorliegen eines bestimmten Berechtigungszustands ausgegeben werden darf;
- Ausgeben eines entsprechenden Warnsignals (254) falls dieser Berechtigungszustand aktuell nicht vorliegt.

12. Verfahren gemäß mindestens einem der Ansprüche 9 bis 11, weiterhin aufweisend die Schritte
- Empfangen eines von der Benutzereingabe (124) abhängigen Berechtigungssignals (126);
- Bestimmen eines aktuell vorliegenden Berechtigungszustands basierend auf dem Berechtigungssignal (126), wobei für eine vorbestimmte Teilmenge von Datenarten eine Ausgabe der entsprechenden Datensatz-Daten (142) über das zweite Anzeigesignal (114) nur erfolgt, wenn aktuell ein diesen Datenarten zugeordneter Berechtigungszustand vorliegt.

13. Verfahren gemäß mindestens einem der Ansprüche 9 bis 12, wobei das Verfahren zum Anzeigen einer Ausgabe einer Mehrzahl von elektromedizinischen Geräten (105, 105', 105", 105''') ausgestaltet ist, mit dem zusätzlichen Schritt
- Empfangen von Geräte-Daten (107), die mindestens ein weiteres elektromedizinisches Gerät (105', 105", 105'") betreffen, wobei der den Geräte-Daten (107) zugeordnete Visualisierungstyp eine Geräteinformation umfasst, die das zu den Geräte-Daten korrespondierende elektromedizinische Gerät (105, 105', 105", 105'") indiziert.

14. Programm mit einem Programmcode zur Durchführung des Verfahrens (700) gemäß mindestens einem der Ansprüche 9 bis 13, wenn der Programmcode auf einem Computer, einem Prozessor oder einer programmierbaren Hardwarekomponente ausgeführt wird.
